# EUROPEAN PATENT APPLICATION

(11) **EP 1 647 272 A1**
(43) Date of publication of application: **19.04.2006**
(21) Application number: 04748950.5
(22) Date of filing: 01.07.2004
(51) Int. Cl.: A61K 31/185, A61K 31/19, A61K 31/095, A61P 25/32

(54) **CURING AND PROPHYLACTIC AGENT APPLIED DURING THE USE OF ALCOHOL AND PSYCHOACTIVE SUBSTANCES**

(30) Priority: 02.07.2003 RU 2003119563
(71) Applicant: Zenovich, Sergei Mikhailovich, Moscow, 121170 (RU); Strelets, Boris Khaimovich, St. Petersburg, 199397 (RU)
(72) Inventor: Zenovich, Sergei Mikhailovich, Moscow, 121170 (RU); Strelets, Boris Khaimovich, St. Petersburg, 199397 (RU)
(74) Representative: HOFFMANN EITLE
(86) International application number: PCT/RU2004/000253
(87) International publication number: WO 2005/002564

(57) **Abstract**

The invention relates to medicine and is directed at developing means and methods for narcological practice and for preventive measures in order to reduce the risk of falling sick with such menacing modem diseases like dependence diseases, in particular alcoholism, narcomania and toximania. Said invention provides for using already known chemical compounds, i.e. vicinal dithioglycols for developing medical agents exhibiting properties for preventing contraction or development or reducing the speed of pathological processes forming a base for dependence diseases, in particular withdrawal syndromes. The inventive substances reduce or block pharmacological effects resulting from ethanol injection and/or narcotics, thereby directly influencing pathogenic mechanisms of the dependence diseases, in particular alcoholism It is stated that said agents are particularly efficient when they are perorally administered. Said invention is accompanied by experimental material which proves the large perspective of the inventive agents and of a prophylactic method which provides for the use thereof before and after consuming alcohol-containing beverages or narcotics. The use of the invention makes it possible to reduce the degree of falling sick with alcoholism and narcomania and to reduce the toxic action of alcohols during the household use thereof.

## Description

The present invention relates to medicine and is concerned with medicaments used in narcological practice as well as for prophylactic purposes.

The spread of alcoholic and narcotic dependence is a serious social threat and comprises today a complex of medico-social problems. According to the WHO data, from the number of persons intaking alcohol about 6% fall sick and only 5―6% of them later make complete recovery.

The number of persons indulging in non-medical use of narcotic agents as such amounts to millions. High level of household use of ethanol, factors of social and economic character, environmental customs lead to tens of millions of people episodically or systematically abusing alcohol, this being the main prerequisite for acquiring certain signs of the dependence syndrome.

It is known that treating patients with the syndrome of alcoholic or narcotic dependence has a restricted effect, because a remission lasting for more than one year after the treatment in a medical hospital is registered only a few percent of patients. Therefore it is expedient to pay greater attention to developing means and methods of treating (acting upon), directed to the prevention of diseases associated with the dependence or directed to reducing or blocking those pharmacological effects of introducing ethanol or psychoactive substances, as a result of whose action the dependence is formed. The point in question is to act upon the pathogenetic mechanisms of development of alcoholic or other dependence on psychoactive substances.

The present invention is directed to the provision of means for solving problems in the given field.

At present there have been developed and are offered for use various medicamentous means for treating and preventing alcoholism, narcomania, as well as various pathologies associated therewith.

One of means for combating dependability on alcohol or other toxic substances is a homeopathic preparation produced by corresponding dilutions of the toxic substance proper (RU 2148993 C1, 2000).

It is also known to use preparations based on medicinal plants, for example, an agent "Narkoton" which displays tonic, cleaning and psychosomatic effect upon intake (RU 2142289 C1, 1999), and also antioxidants, for example, mexidol (RU 2145855 C1, 2000), as well as agents arresting intoxication, for example, a complex of pycamylon, lipoic acid, pyridoxine and panthenol (RU 2165759 C1, 2001).

A preparation for eliminating drug abuse and intoxication by chemical substances is known in the art, which is based on sulfur-containing compounds, comprising sodium thiosulfate, vitamin C, magnesium sulfate, and sodium bicarbonate (RU 2108101 C1, 1998).

The above-cited agents differ in their chemical nature are intended for the most part for eliminating certain symptoms originating upon intake of toxic substances, alcohol, psychoactive substances. However, these agents do not effectively prevent the development of alcoholism and/or drug abuse.

It is an object of the present invention to bring into circulation an effective agent, without side effects or with minimized side effects, for preventing the origination and/or blocking and/or reducing the rate of development of pathological processes induced by both single and repeated, particularly long-term, systematic use of alcoholic beverages, of household-grade alcohol, of substances having addictive potential.

The stated object is accomplished by the provision of an agent for oral or nasal administration, which comprises at least one vicinal dithioglycol of general formula (1), incorporated, if necessary, into a pharmaceutically acceptable. The term vicinal dithioglycol is used to denote substances of the general formula

R₁CH(SH)(CH(SH)R₂ (1)

in which R₁ stands for radicals: (―H) or (―COOH) or (―SO₃H),
R₂ stands for radicals: (―H) or (―COOH), or (―OH), or (―CH₂―O―CH₂―SO₃H), or (―CH₂―COOH), or (―CH₂―OH), or (―CH₂―SO₃H), or salts of their derivatives, containing salt-forming groups (―OH) or (―COOH), or (―SO₃H).

Compounds of general formula (1) are known from the state of the art. Their description, routes to the synthesis are presented, e.g., in "Khimicheskaya Entsiklopediya" ("Khimicheskaya Entsiklopediya" in 5 volumes, Moscow: Sovetskaya Entsiklopediya Publishers, 1990, vol. 2, pp. 91―92).

To vicinal dithioglycols there belong, in particular:
1. Dithioglycerine ("Dicaptol ","Dimercaprol", BAL-British Anti-Lewisite) having the formula: 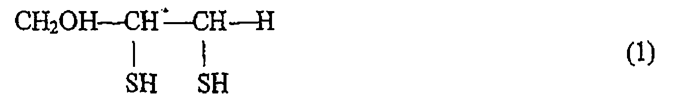
2. 2,3-Dimercaptopropane-sulfonate Na (DMPS, "Unithiol", "Dimaval") having the formula 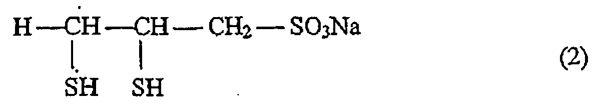
3. 2,3-Dimercaptosuccinic acid (DMSA, "Succimer") having the formula: 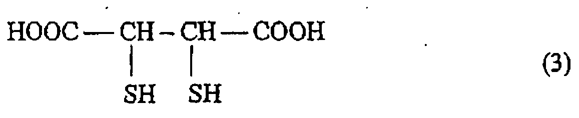
4. 2-(2,3-Dimercaptopropoxy) ethanesulfonate Na ("Oxathiol") having the formula:

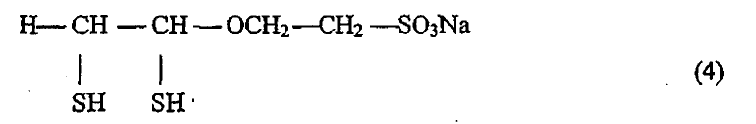

Vicinal dithioglycols are used as medicaments having antiradiation activity, and also as antidotes in cases of poisoning with heavy metals and their derivatives, particularly in cases of poisoning with arsenic, lead, mercury (Mashkovsky M.D., "Drugs" in 2 volumes, Moscow: Meditsina Publishers, 1988, vol. 2, pp. 181―183). These are low-molecular compounds with two adjacent mercapto groups, due to which they are a good trap for heavy metals, including radio nuclides

In narcological practice it is known to use Unithiol in cases of alcoholic delirium, acute alcoholic intoxication, narcotic intoxication, as an auxiliary component in complex therapy together with other detoxicants (Mashkovsky M.D., "Drugs", Moscow: Meditsina Publishers, 1993). It is known to use Unithiol in arresting withdrawal syndrome as one of the steps in carrying out desintoxication therapy (G.V. Morozov (Ed.), "Alcoholism", Moscow: Meditsina Publishers, 1985, pp. 314―321). According to the cited purposes, these compounds to the present day have been used only in injection form, always as an adjuvant.

We were the first to find out that vicinal dithioglycols upon oral administration display an unexpectedly higher effect in the case of the above-cited known purposes, and also in the case of broader purposes. More than that, we have unexpectedly found out that administration, and especially oral administration, of dithioglycols gives a novel effect which manifests itself first of all in blocking and/or inhibiting pathological processes that originate as a consequence of using ethanol-containing beverages and/or substances having an addictive potential, such as psychoactive or psychotropic substances.

The proposed agent brings about the following effects which manifest themselves first of all in connection with drinking household-grade ethanol or indulging in alcohol-containing beverages, and also in connection with using psychoactive substances (PASs):
- decreasing the rate of dependence formation;
- decreasing the rate of withdrawal syndrome formation;
- blocking or inhibiting the development of somatovegetative symptoms of withdrawal syndrome;
- blocking or inhibiting the development of psychoneurological symptoms of withdrawal syndrome;
- blocking or inhibiting the formation of morbid attraction to alcohol and/or PASs;
- blocking or inhibiting the formation of the quantitative control loss symptom;
- inhibition of tolerance development;
- inhibition of the development of organ pathologies against high tolerance background;
- inhibition of the development of somatoneurological disorders against high tolerance background;
- inhibition of the development of vegetovascular, veretoasthenic disorders against high tolerance background;
- inhibition of the development of psychopathy-like or psychic disorders against high tolerance background;
- reducing the reinforcing properties of ethanol and/or other ingested PASs;
- reducing the rate of development of organ pathologies, particularly of the brain, liver, myocardium;
- reducing the rate of development of somatoneurological disorders;
- inhibition of the development of psychopathy-like or psychic disorders;
- reducing the rate of lowering of cognitive-mnestic functions, in particular, preventing the origination of alcoholism amnesia.

Since we have discovered novel properties of the compounds of general formula (1), one more aspect of the present invention is use of at least one vicinal dithioglycol as an agent preventing the development of alcoholism (alcohol dependence syndrome according to the WHO terminology) and/or dependence on the intake of substances having an addictive potential, such as psychoactive substances. This purpose is novel, it is not based on the known desintoxicative, antioxidant properties of these substances.

We have revealed novel properties of said substances, owing to which their effect for a new purpose manifests itself.

It is known that a common link in the pathogenesis mechanism when intaking ethanol and/or narcotic agents is a disturbance in the system of neuromediatory regulation, particularly, in the catecholamine system Upon acute intoxication with ethanol a change in the functions of the central nervous system (CNS) is caused both by ethanol as such and by its metabolites, first of all by acetaldehyde. They enhance the release of catecholamines from adrenergic endings. Acetaldehyde can enter into reaction of condensation with catecholamines, particularly with dopamine, giving tetrahydroisoquinolines (THIQs). One of THIQs, salsolinol, is a hallucinogen leading to the development of withdrawal syndrome. Condensation of acetaldehyde with serotonin leads to the formation of harmaline which is also a strong hallucinogen. Accumulation of THIQs in brain neurons, which behave as "false mediators" instead of catecholamines, leads to activation of opiate receptors, lowering of the enkephalin synthesis and to the development of withdrawal syndrome, alcoholism, dependence

Administration, including and especially, preventive administration of vicinal dithioglycols leads to blocking or inhibiting the process of formation of THIQs and, consequently, to the same effect on the process of formation of withdrawal syndrome and dependence syndrome, and also on other consequences of the appearance of THIQs.

The dopaminergic system of the brain plays an important role in the genesis of various psychic disorders. It has been proved that dopamine participates in control of the reinforcing mechanisms of the brain, in particular, of hypothalamic self-stimulation effects. It has also been proved that calcium also participates in the effects produced by ethanol on dopaminergic neurons.

Acute effects of ethanol are mediated also by the neuromediatory system of glutamic acid (glutamate), mainly via N-methyl-D-aspartate (NMDA) receptors. Ethanol alters the activity of the glutamatergic system, by inhibiting said activity. NMDA receptors participate in the formation of memory traces due to long-term postsynaptic potentiation of synapses. The NMDA receptor is connected with the calcium channel and its excitation enhances the permeability of the cell membrane for new calcium ions. Ethanol even in low doses causes a reduction of ionic currents and suppresses NMDA-stimulated capture of calcium and also blocks long-term potentiation induced by calcium

A rise of the level of intracellular calcium (which can be mediated by the NMDA receptor activity) is also regarded as modulation of the activity of the "GABA receptoi-/chloride channel" complex (GABA denoting γ- aminobutyric acid). It is believed that the GABAergic neuromediatory system responsible for the inhibition of excitation is also a target for the toxic action of ethanol. These systems are modulated by the level of calcium ions.

It is known that the GABAergic system participates in the mechanism of formation of alcoholic dependence and ethanol withdrawal phenomenon, particularly due to the GABA-mediated current of chlorine ions. Abuse of ethanol, particularly its systematic or chronic consumption leads to the maladjustment of conductance in the GABAergic neurons, to a change in the properties of the GABA receptors and ionic currents. It has been established that cAMP (cyclic adenosine monophosphate) system and magnesium ions are responsible for maintaining the normal level of functioning of the GABAergic system. The responsibility of the maladjustment phenomena of the NMDA receptor system and of calcium channels associated therewith in the genesis of the withdrawal syndrome, in an increase of nervous irritability and in other neurological and psychic disorders has also been established.

Unfavorable effect of ethanol and other PASs on the system of neurohormonal regulation is also known. Neurohormones may change the effectiveness of the afferent input or promote switching thereof from one channel of multisensory input to another, i.e., modulate changes in the functional condition of large populations of brain cells. This is the basis for regulation of behavioral processes, changes in the effectiveness of reinforcing systems, regulation of affective states, learning and memory traces consolidation processes. Release of some neurohormones is calcium-dependent, as well as dependent on the state of the biomembrane.

The action of many other PASs on the brain occurs, in particular, through the interaction of PASs or their metabolites with receptor proteins of the biomembranes of nervous tissue cells, and also with other membrane structures playing the role of functional pores for the transport of substances, including ions, particularly metal ions. Some metals, such as calcium, magnesium, play an important role in modulating neuron responses to signals.

Vicinal dithioglycols significantly influence calcium exchange, thereby facilitating the transport of calcium ions through membranes. They also influence the processes of calcium release and accumulation in intracellular depots. Owing to this action, VDs are capable of changing the unfavorable action of ethanol and/or other PASs on calcium metabolism, which unfavorable action affects the processes of excitation and inhibition in synapses and then leads to dysfunctions of the CNS, particularly, to memory impairments, amnesia and more grave cognitive-mnestic and behavioral disorders. VDs possess the same property with respect to magnesium ions, which also makes it possible to reduce the level of the pharmacological effects in administering ethanol and/or PASs.

Many pharmacological effects in administering ethanol and/or other PASs are caused by their action on the properties and structure of the biological cellular membrane. Here we deal with changes in the physical and physicochemical properties of the lipid bilayer, in particular with an increase of its fluidity. A disturbance of the fluidity on acute injection of ethanol and/or PASs disturbs the normal course of nervous processes. The process of the cellular membrane adaptation to a change in the fluidity in systematic intoxication leads to a reduction of the fluidity effect, this being one of the constituent mechanisms of tolerance and dependence formation, underlying the development of alcoholism and narcomania.

The major factor behind toxic damage of biomembranes in intoxication with alcohol and/or other PASs is the mechanism of free-radical oxidation of their lipid component with simultaneous reduction of the antioxidant resistance of the organism. Compounds containing SH-groups, together with uric acid, vitamins E, A, ascorbic acid, belong to the category of basic endogenous antioxidants, because it is just they that play the main role in the protection of biomembranes in oxidative stress. Pharmacological effects of administering ethanol and/or PASs reduce the activity of natural antioxidants, particularly of those containing SH-groups, toxic metabolites quickly deplete their stock, and the oxidative stress gains development. Frequent or episodic but strong intoxications lead to irreversible destruction of the biomembrane and cell death, the process of degradation of the nervous tissue being thus intensified. The myelin sheaths of axons also gradually degrade in accordance with the free-radical oxidation mechanism, and this leads to axon neuropathies, to the effects of ephaptic transmission of excitation, and to building-up of psychopathological processes and neuropathologies.

Vicinal dithioglycols are capable of stopping the process of free-radical destruction of the biomembrane, in particular they protect the myelin sheaths of axons from damage, protect hepathocytes, vessels, brain structures. On the strength of the above-indicated properties of vicinal dithioglycols, their administration makes it possible to block or reduce the level of damaging effects on the myocardial tissue, on the pancreas, on the vascular system and other organs and systems of the organism

These and other actions of vicinal dithioglycols on processes associated with pharmacological effects of administering ethanol and/or other PASs condition the presence of the effects which are cited above and are technical results of the claimed solution.

Toxic effects of administering ethanol and many other psychotropic substances, for example, opioid substances, cannabinoids, and the like, manifest themselves by the effect they produce on many organs and systems of an organism, i.e., lead to simultaneous triggering or activating quite a number of pathological processes. Taking into account the above-said properties of the vicinal dithioglycols, it can be asserted that they favorably act on quite a number of such pathological processes, but this action may be manifested or realized, though simultaneously, but with different intensity. The degree of intensity of action on a particular pathological process depends, first of all, on the stage in the process history. The origination of some processes can be blocked by preventive administration of the agents according to the invention, while other already commenced pathological processes can be inhibited by intaking these agents. The intensity of action on a particular pathological process caused by the toxic effects of administering ethanol and/or other PASs depends also on the dose of the inventive agent, on somatic and other particulars of an individual, on the tactics of intaking the agent, on the toxic load, and on other factors.

We have discovered that the above-indicated physiologically favorable effects manifest themselves, and manifest themselves with particular strength, in oral administration. A good effect takes place also in topical administration via the oral mucosa, in nasal administration, and anapnotherapy. Apparently, this is associated with a high penetration rate of the substance into the liver and with particulars of the substance interaction with liver enzymes.

The effects indicated by us herein-above broaden substantially the heretofore-known properties of the vicinal dithioglycols. Their known detoxification activity was used for arresting acute withdrawal conditions, i.e., for treating humans affected with alcoholism. Treatment with Unithiol in cases of delirium tremens is carried out with respect to patients suffering from alcoholism in the terminal stage of the disease, which is characterized by the presence of a serious psychic disorder. The known antioxidant activity also favorably complements the novel potentialities of the vicinal dithioglycols. Earlier we revealed the ability of the vicinal dithioglycols to alleviate the severity of household-level hangover, the latter being understood as the unfavorable consequences of the use of ethanol that take place during the first twenty-four hours after its use. This effect is conditioned by the activity of the substance with respect to carbonyl compounds (RU 2157647 C1, 2000). At present, basing on the effects again found out in experiments, and on theoretical interpretation thereof, one can assert that the effects of the vicinal dithioglycols as of the active substance manifest themselves when the vicinal dithioglycols are administered in isolation from the fact of using ethanol, for instance, when they are administered preventively several weeks before (course intake) or several days or hours before the use of ethanol, as well as twenty-four hours or more after the use of ethanol. The same holds true for other agents having an addictive potential. It is important to point out that we have established the action of the vicinal dithioglycols on common links of the pathogenesis of dependences of different etiology, not associated with administering namely ethanol, but merely a psychotropic substance of various origin.

Therefore, there is every reason to draw a conclusion about the synergism of the known properties of the vicinal dithioglycols with the above-indicated properties which allow the vicinal dithioglycols to be recommended for use for the new purpose.

The pharmaceutical form of the proposed agents can comprise forms suitable for oral, nasal, topical (including transbuccal and sublingual) administrations via the oral mucosa or for administering by inhalation. The pharmaceutical forms can comprise compositions, be represented by convenient discrete dosed units, and be prepared by any methods well known in pharmacy. All these pharmaceutical methods comprise a step of combining the active substance with liquid vehicles or with finely divided solid excipients or with both, if required, followed, if necessary, by shaping the product in a desired form.

Pharmaceutical forms suitable for oral administration can be discrete units such as tablets, cachets, starch capsules, each containing a prescribed amount of an active ingredient; or in the form of powders or granules; or in the form of a solution, suspension or emulsion. The active ingredient can also be represented in the form of a bolus or paste or be in the pure form, i.e., without a vehicle. Tablets and cachets for oral administration, besides additional physiologically active components, can also contain commonly adopted additives, such as binders, fillers, lubricants, aerators, wetting agents. Tablets can be produced by pressing or casting in molds, optionally, with one or more additional ingredients. Pressed tablets can be manufactured by pressing the active ingredient in suitable machines in a free-flowing form such as powder or granules, optionally mixed with a binder, lubricant, an inert diluent, lubricating agent, surfactant or dispersant. Cast tablets can be prepared by casting a mixture of pulverized compounds, wetted with an inert liquid diluent, into molds in a suitable machine. Tablets can be coated with an envelope in accordance with methods well known in this field of the art. Liquid oral preparations can be in the form of, say, aqueous or oil suspensions, solutions, emulsions, syrups or elixirs, or can be represented in the form of dry products for mixing before use with water or other suitable solvent. Such liquid preparations, besides additionally introduced physiologically active components, can contain commonly adopted additives, such as suspending agents, non-aqueous solvents (which can comprise edible oils), flavorings, colorants or preservatives. The above-cited forms can be optionally manufactured so as to provide slow or controlled release of the active substance contained therein.

The forms for topical administration via the oral mucosa, e.g., transbuccally or sublingually, comprise lozemges containing an active ingredient in a corrigent base such as a carbohydrate, e.g., sucrose, acacia or tragacanth, and pastils containing an active substance in such a base as gelatin, glycerin, a carbohydrate, acacia, etc. For intranasal administration of the above-said active substances such forms can be employed as an aerosol or sprayed powder or drops. Drops can be formulated on an aqueous or non-aqueous base containing one or more dispersants, solubilizing agents or suspending agents. Liquid aerosols can be conveniently administered from pressurized packages. For administering by inhalation, it is convenient to introduce the compounds according to the present invention from insufflators, inhalation pressurized packages or other convenient means of obtaining aerosol sprays. Pressurized packages can contain a suitable propellant, such as dichlorodifluoromethane, trichlorofluoromethane, carbon dioxide or other suitable gas. In the case of a pressurized aerosol the dosage unit can be determined by providing a valve for delivering a measured amount.

Alternatively, for administering the substances according to the invention by inhalation or insufflation, it is possible to use dry powder-like formulas, for instance, a powder-like mixture of an active substance and a suitable powder-like base, such as lactose, starch, talc. The powder-like composition can be in the form of a single dose, e.g., in capsules, cartridges, gelatin or plaster packages, from which powder can be introduced with the aid of an inhalator or insufflator.

If necessary, the above-described forms can be used, adapted for providing continuous release of the active ingredient.

The pharmaceutical agents according to the invention can also contain one or more active ingredients, adding of which, as a rule, serves to enhance the action of the substance of the invention and also can serve for attaining synergism of the action of the added ingredients and of the compounds of the invention. The added active ingredients can be those selected from the group comprising a vitamin, a vitamin precursor, a vitamin derivative, an organic acid, an organic acid derivative, a lipid, a physiologically active peptide, an amino acid, an amino acid derivative, an enzyme, an enzyme derivative, an enzyme precursor, a coenzyme, a coenzyme derivative, a coenzyme precursor, a carbohydrate, a protein, and mixtures thereof. For example, it can be favorable to add amino acids that are neuromediators, such as glycine, γ-aminobutyric acid (GABA). It is favorable to add sources of metal ions, because introducing methanol and some other PASs disturbs the water-electrolyte balance. It is also favorable to add sources of divalent metal ions, because some of these metals, such as magnesium, calcium, manganese, play an important role in enzymatic catalysis and enter into the formulation of coenzymes. Shortage of calcium which is a modulator of a number of processes, as stated above, and also toxic suppression of its metabolism, accelerates the development of many pathological processes. Favorable additions in the formulation of the agents according to the invention can be also sources of lipids, particularly of phospholipids, e.g., lecithin, of glycolipids, and other hepatoprotectors, particularly vitamins, e.g., α-tocopherol, their precursors, their derivatives, physiologically active peptides, e.g., neuropeptides, as well as many other known physiologically active substances.

It should be understood that besides the ingredients partly cited above, the agents of the invention can comprise other ingredients, agents conventional for the given field of application and employed for the form of the agent particularly dealt with. For example, the agents for oral administration can comprise flavoring or structure-forming agents.

Preferable forms with fractional dosages are those comprising an effective dose of the agent according to the invention, as is set out below, or a suitable fraction of the indicated dose.

For accomplishing the recommended objects according to the invention, the above-said agent comprising an active ingredient in an effective amount can be administered orally or by other convenient route in a dose of from 0.1 to 250 mg/kg (per kilogram of the recipient's weight) per day. The dose level for a normal adult human usually ranges from about 5 mg to about 10 g per day, as a rule, within still narrower limits of from about 50 mg to about 2 g per day, most preferably from 100 to 1000 mg per day. Tablets or other forms of preparing the agent, represented in the form of discrete units, can for the sake of convenience contain an amount which is effective in such dosage, or a multiple amount, e.g., units containing from 50 to 300 mg, usually about 100―250 mg.

The agent according to the invention is administered preferably per os, the exact amount of the agent, correspondingly, doses of the agent, should be taken in accordance with the instruction for use or recommendations of a specialist. However, the actually used dose will depend on many factors, including sex, age, body weight, risk factors associated with the conditions of taking alcohol and/or PASs, somatic and psychoneurological particulars of an individual, etc. Factors in the same context must be taken into consideration when determining the time of administration and duration (frequency) of administration of the agent. The agent can be recommended for administration in the form of a course ,it can be prescribed before the use of an alcoholic beverage and/or PAS(s), or after a definite period of time after such use, or before and after such use, or in other combinations with the use of ethanol or PAS(s). Both the above-cited and other possible variants regulating the temporal relationship between the moment(s) of administering the agent and the moment(s) of using ethanol and/or PAS(s) can partially overlap.

The examples given below illustrate the essence of the invention but do not limit the scope of the set of claims.

### Example 1. Unithiol tablets

There are mixed 25 g of Unithiol (powdered substance), 5 g of lactose, 10 g of aerosil, 30 g of starch, 5 g of starch glucolate sodium salt, 5 g of magnesium stearate, 1 g of calcium pantothenate, 7 g of folic acid, 7 g of ascorbic acid, the mixture is moistened, thoroughly stirred, homogenized and tabletted to obtain tablets, 0.5 g each.

| **Formulation of tablets,** | **weight %** | **mg** |
|---|---|---|
| Unithiol | 30 | 150 mg |
| Lactose | 5 | 25 mg |
| Aerosol | 10 | 50 mg |
| Corn starch | 30 | 150 mg |
| Starch glucolate sodium salt | 5 | 25 mg |
| Magnesium stearate | 5 | 25 mg |
| Calcium pantothenate | 1 | 5 mg |
| Folic acid | 7 | 35 mg |
| Ascorbic acid | 7 | 35 mg |
| Wetting agent | the balance | |

### Example 2. Quick-dissolving capsules filled with granules comprising:

| | |
|---|---|
| Dicaptol | 50 mg |
| Succimer | 50 mg |
| Unithiol | 100 mg |
| Succinic acid | 100 mg |
| Calcium citrate | 100 mg |
| Lecithin | 50 mg |
| Magnesium oxide | 50 mg |
| Pyridoxine hydrochloride | 20 mg |

Granules were produced by following a technology known in pharmacy

The possibility of use for the above-stated purposes was tested on laboratory animals and in clinical practice on volunteers. Investigations of the preventive and protective properties of the vicinal dithioglycols (on an example of Unithiol) were carried out on the initiative of the authors jointly with the National Narcology Research Center of the RF Ministry of Health and Moscow Research-and -Prophylaxis Center for Preventing Narcomania at the Health Care Committee of the City of Moscow.

**Example 3.** An experimental part of the work was carried out on male rats of the Wistar-100 line with the initial body weight of 150―250 g. The animals were kept under artificial illumination conditions (12 hours a day) and constant access to standard formula feed in water. Each group of the animals consisted of 10 individuals.

For modeling acute intoxication with narcotic analgesics, the rats were administered once intraperitoneally with morphine hydrochloride in a dose of 25 kg/kg body weight.

Chronic intoxication with narcotics was modeled by administering animals intraperitoneally with morphine hydrochloride during 7 days in uniformly increasing doses of from 10 to 70 mg/kg animal body weight. Control groups (CGs) of the animals were administered with physiological solution in the same volumes.

20 minutes after administering narcotics to the animals, both under the conditions of acute experiment and in chronic experiment, the animals of the first test group (TG-1) received Unithiol per os in a gelatin capsule (15 mg/kg body weight). The animals were decapitated 2.5 hours after the acute administration or ― under the conditions of chronic experiment ― after the last administration of morphine (the 7^{th} day of chronic experiment).

Chronic alcohol intoxication was modeled by affording free access for the animals to a 12% solution of ethanol (the only source of liquid) during 30 days. A part of the animals (the first test group, TG-1) along with the ethanol solution received once Unithiol in an amount of 25 mg/kg animal body weight at the end of the experiment; the second part of the animals (the second test group, TG-2) received instead of Unithiol an empty capsule. The control group (CG) had free access to drinking water as the source of liquid.

The results of the experiment on animals showed that the concentration of the end products of the peroxidation of lipids ― TBA-positive compounds (according to thiobarbituric test) in the blood plasma of both chronically morphinized and chronically alcoholized individuals, takes up values reliably differing from an analogous group without use of Unithiol toward ;lowering. The content of the main endogenous antioxidants ― vitamins E, A, SH-groups in the case of oral administration of Unithiol was preserved on the level close to the values in the control group.

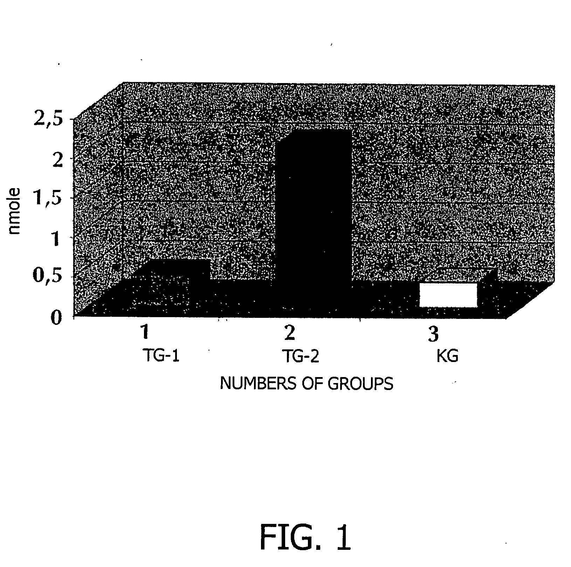

Fig. 1 shows data in the form of columns, which characterize the levels of lipid peroxides in each test group of the animals in acute intoxication with morphine.

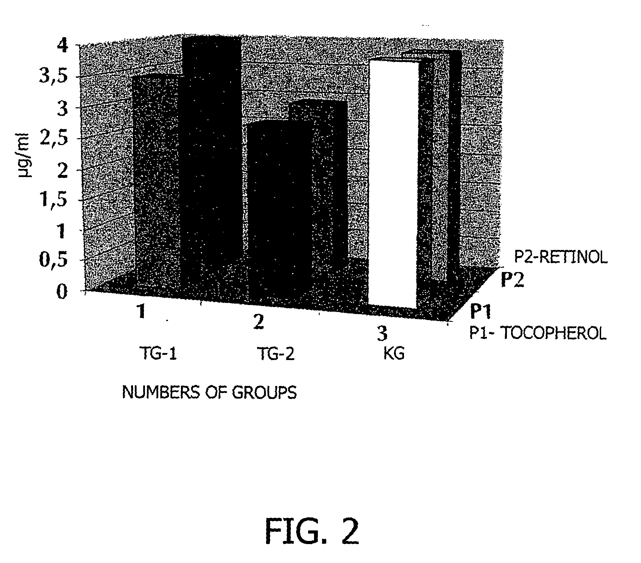

Fig. 2 shows data about measuring the levels of vitamins E and A in the blood plasma of the rats of each group in the experiment on chronic intoxication with morphine. A comparison of the levels for all the three groups of the animals shows that sweeping down of the level of vitamins A and E occurred in the animals which received only morphine and an empty capsule. The animals which received Unithiol along with morphine preserved the mean content of vitamin E and A on the level of values in the control group. The same situation was observed in changes of other endogenous antioxidants.

The activity of enzymes characterizing damage of hepatocyte membranes (ALT ― Alanine aminotransferase, AST ― Aspartate aminotransferase) also proved to be reliably lower than in the control group (without use of Unithiol). Measurements of the activity of Alanine aminotransferase in the homogenates of the rats' liver in the experiment in the case of chronic intoxication with morphine showed that the ALT activity in the rats which received Unithiol in parallel with morphine was twice lower than in the animals which received morphine and an empty capsule.

In another fragment of the experiment on chronic introduction of ethanol the difference in the routes of administering Unithiol was studied. One group of chronically alcoholized rats received once Unithiol in a capsule *per os*, while an other group received once the same dose of Unithiol (25 mg/kg body weight) by injection. The animals were decapitated 2.5 hours after the administration of Unithiol. Using liver tissue homogenates, a comparative study of the content of lipid peroxides (TBA-positive products), triglycerides, of the activities of enzymes-aminotransferases ALT, AST, was carried out. To unify the comparison for each characteristic, the level of the characteristic for the group of the animals which received Unithiol *per os* was adopted for 100%.

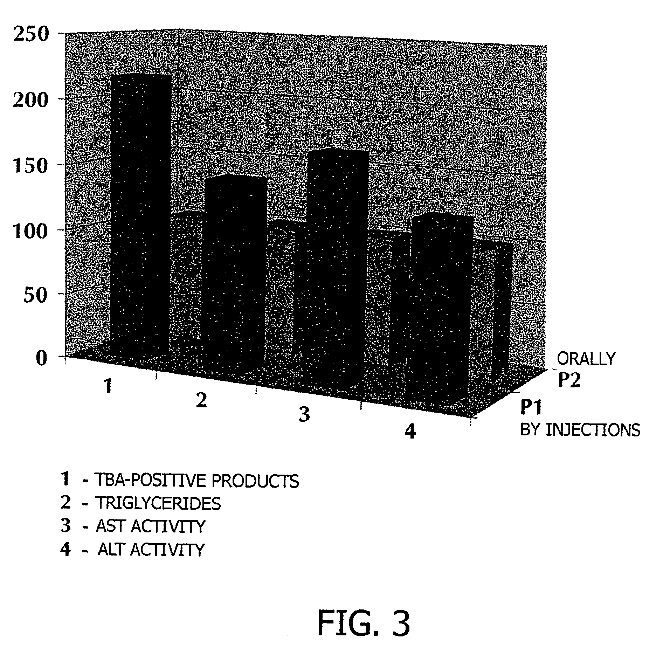

Fig. 3 shows graphic data of the comparison of these characteristics. These data demonstrate that oral administration of Unithiol yields essentially better values of the biochemical parameters characterizing the extent of toxic action of ethanol on the liver tissue than administration by injection. These differences are statistically significant.

The second fragment of the preventive, protective and antitoxic properties of Unithiol was studying its influence in "per os" application on the condition of volunteers after intaking a low-alcoholic beverage (beer).

**Example 4.** For an experiment on volunteers, 3 groups were created: one control group and two test groups. The participants in the experiment were adult volunteers of both sexes, who had no chronic diseases in the anamnesis and who did not abuse alcoholic beverages. The experiment lasted two weeks. Alcoholic intoxication was created by regular (during 2 weeks) nightly intake of one liter of low-grade beer having a large amount of aldehydes. Additional intake of alcoholic beverages by the test subjects was not limited. During the first week the first test group (TG-1) received an alcoholic load in the evening and Unithiol in the morning, in the amount of 250 mg per os in the form of an aqueous solution. During the second week instead of Unithiol the first test group received in the morning 250 ml of drinking water, the evening alcoholic load was preserved. The second test group (TG-2), on the contrary, with an analogous alcoholic load in the evening, received 250 ml of water during the first week of the experiment, and a solution of Unithiol in the morning during the second week. Such design of the experiment allowed revealing the preventive possibilities of Unithiol with a sufficiently high level of toxic alcoholic load. The test subjects were under every day medical supervision. Biochemical investigations of biological fluids of the test subjects were also carried out for assessing the rate of the lipid peroxidation (LPO) and the condition of the endogenous antioxidant resistance system of the organism The concentrations of the POL products (TBA-test) ― of malonic dialdehyde (MDA), the level of vitamins E and A, of SH-groups, as well as the activity of enzymes-liver pathology markers (ALT, AST) and of triglycerides were measured.

In the tests it was found that peroral use of Unithiol against the background of withdrawal removes the subjective perception of withdrawal 8―10 minutes after the intake of the preparation. The intake of water did not alleviate the condition. Objective biochemical values also testify to the effect produced by the preparation on the test subjects. Statistically significant differences between the same values against the background of alcoholic intoxication without the intake and with the use of the preparation are noted for all the participants in the tests. The data were treated statistically by nonparametric statistical methods.

All the biochemical values measured in the tests statistically significantly worsened for the participants in the test groups on ingesting alcohol without Unithiol. In the first test group taking Unithiol during the first week against the background of evening use of alcohol did not lead to worsening of the biochemical values, whereas during the second week, when taking the preparation was replaced with taking water, the values significantly worsened. A one-week taking of alcohol followed by a one-week taking of alcohol and Unithiol (TG-2) led to the restoration of the initial (basic) level of the biochemical values in the test subjects.

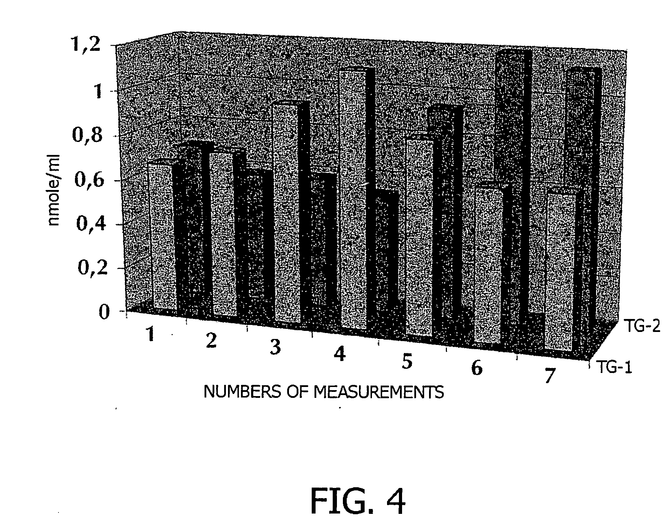

Fig. 4 shows MDA data for both groups of the test subjects. Measurements were carried out for urine every 2 days during two weeks of the experiment.

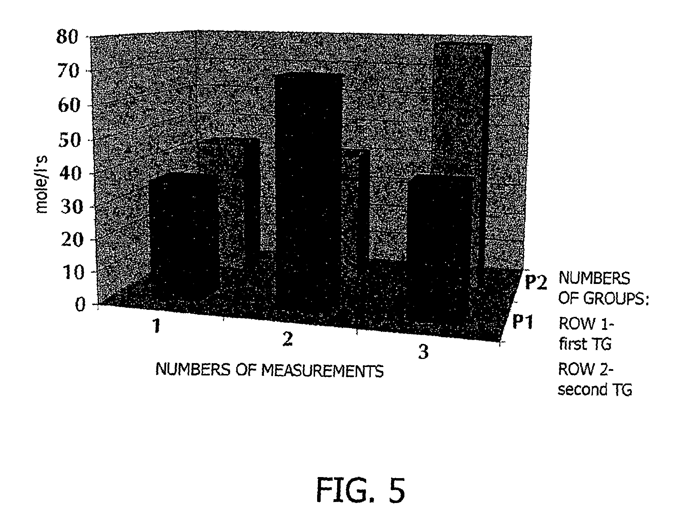

Fig. 5 shows data about the activity levels of the measured amino transferases (on the example of AST) for both groups of the test subjects. Measurements were carried out three times: at the beginning of the tests, in a week, 2 weeks after the beginning of the experiment.

The obtained data convincingly testify to the systemic preventive effect of Unithiol, which manifests itself in blocking the development of the withdrawal syndrome, in inhibiting and even blocking the toxic effect on the liver parenchyma, in blocking peroxidation processes and, consequently, in preventing the development of the processes of toxic action of ethanol.

## Claims

1. An agent for preventing the origination and/or preventing the development and/or reducing the speed of the development of pathological processes induced by using alcohol-containing beverages and/or substances having an addictive potential, **characterized in that** it comprises as at least one active component at least one vicinal dithioglycol of the general formula
R₁CH(SH)(CH(SH)R₂ (1)
in which R₁ stands for radicals: (―H) or (―COOH) or (―SO₃H),
R₂ stands for radicals: (―H) or (―COOH), or (―OH), or (―CH₂―COOH), or (―CH₂― OH), or (―CH₂―SO₃H), or (―CH₂―O―CH₂―SO₃H),
or salts of their derivatives, containing salt-forming groups (―OH) or (―COOH), or (― SO₃H),
in an effective amount in a form suitable for oral or nasal administration.

2. The agent according to claim 1, **characterized in that** the substance having an addictive potential is a narcotic substance.

3. The agent according to any one of claims 1, 2, **characterized in that** it is used for attaining at least one effect selected from the group consisting of
- decreasing the rate of dependence formation;
- decreasing the rate of withdrawal syndrome formation;
- blocking or inhibiting the development of somatovegetative symptoms of withdrawal syndrome;
- blocking or inhibiting the development of psychoneurological symptoms of withdrawal syndrome;
- blocking or inhibiting the formation of morbid attraction to alcohol and/or PASs;
- blocking or inhibiting the formation of the quantitative control loss symptom;
- inhibition of tolerance development;
- inhibition of the development of organ pathologies against high tolerance background;
- inhibition of the development of somatoneurological disorders against high tolerance background;
- inhibition of the development of vegetovascular, vegetoasthenic disorders against high tolerance background;
- inhibition of the development of psychopathy-like or psychic disorders against high tolerance background;
- reducing the reinforcing properties of ethanol and/or other ingested PASs;
- reducing the rate of development of organ pathologies, particularly of the brain, liver, myocardium;
- reducing the rate of development of somatoneurological disorders;
- inhibition of the development of psychopathy-like or psychic disorders;
- reducing the rate of lowering of cognitive-mnestic functions, in particular, preventing the origination of alcoholism amnesia.

4. The agent according to any one of claims 1―3, **characterized in that** it contains a pharmaceutically suitable vehicle.

5. The agent according to claim 4, **characterized in that** said vehicle comprises auxiliary substances selected from the group consisting of a binder, a filler, a lubricant, a disintegrant, a wetting agent, an inert solvent, a surfactant, a dispersant, a suspending agent, an emulsifying agent, an edible oil, flavorings, a food colorant or mixtures thereof.

6. The agent according to claim 4, **characterized in that** it is made in a form for topical administration via the oral mucosa, wherein the above-said vehicle comprises an ingredient selected from the group consisting of a flavoring agent, a carbohydrate, acacia, tragacanth, gelatin, glycerin and mixtures thereof.

7. The agent according to claim 4, **characterized in that** it is made in a form for nasal administration, wherein the above-said vehicle comprises an ingredient selected from the group consisting of a dispersing agent, a solubilizing agent, a suspending agent or mixtures thereof.

8. The agent according to claim 4, **characterized in that** it is made in a form for administration by inhalation, wherein the above-said vehicle comprises a propellant.

9. The agent according to any one of the preceding claims, **characterized in that** the amount of a vicinal dithioglycol of general formula (1) is from about 5 mg to about 10 g per day.

10. The agent according to claim 9, **characterized in that** the amount of a vicinal dithioglycol of general formula (1) is from about 50 mg to about 2 g per day.

11. The agent according to claim 10, **characterized in that** the amount of a vicinal dithioglycol of general formula (1) is from about 100 mg to about 1000 mg per day.

12. The agent according to any one of claims 1―11, **characterized in that** it further comprises at least one source of metal ions for maintaining the water-electrolyte balance and/or normal level of enzymatic activity and/or of neuroregulation and excitation or inhibition transmission systems.

13. The agent according to claim 12, **characterized in that** said metal is a bivalent metal, such as calcium, magnesium, manganese, etc.

14. The agent according to any one of claims 1-13, **characterized in that** it further comprises at least one ingredient selected from the group consisting of a vitamin, a vitamin precursor, a vitamin derivative, an organic acid, an organic acid derivative, a lipid, a physiologically active peptide, an amino acid, an amino acid derivative, an enzyme, an enzyme derivative, an enzyme precursor, a coenzyme, a coenzyme derivative, a coenzyme precursor, a carbohydrate, a protein, and mixtures thereof.

15. The agent according to any one of claims 1-14, **characterized in that** it is intended for preventive or curative-preventive purposes in a condition wherein the origination or development of alcoholism and/or of dependence on psychoactive substances can take place.

16. Use of at least one vicinal dithioglycol of general formula (1) which possesses the property of blocking or inhibiting the formation of tetrahydroisoquinolines (THIQs) for preparing medicaments intended for blocking and/or preventing the development and/or for reducing the rate of development of pathological processes caused by using alcohol-containing beverages and/or substances having an addictive potential.

17. Use of said compounds according to claim 16, **characterized in that** there is attained at least one effect selected from the group consisting of:
- decreasing the rate of dependence formation;
- decreasing the rate of withdrawal syndrome formation;
- blocking or inhibiting the development of somatovegetative symptoms of withdrawal syndrome;
- blocking or inhibiting the development of psychoneurological symptoms of withdrawal syndrome;
- blocking or inhibiting the formation of morbid attraction to alcohol and/or PASs;
- blocking or inhibiting the formation of the quantitative control loss symptom;
- inhibition of tolerance development;
- inhibition of the development of organ pathologies against high tolerance background;
- inhibition of the development of somatoneurological disorders against high tolerance background;
- inhibition of the development of vegetovascular, vergetoasthenic disorders against high tolerance background;
- inhibition of the development of psychopathy-like or psychic disorders against high tolerance background;
- reducing the reinforcing properties of ethanol and/or other ingested PASs;
- reducing the rate of development of organ pathologies, particularly of the brain, liver, myocardium;
- reducing the rate of development of somatoneurological disorders;
- inhibition of the development of psychopathy-like or psychic disorders;
- reducing the rate of lowering of cognitive-mnestic functions, in particular, preventing the origination of alcoholism amnesia.

18. The use according to claim 16 or to claim 17, wherein the vicinal dithiogycol is Unithiol (DMPS).

19. The use of at least one vicinal dithioglycol of formula (1) as an agent preventing the origination or development of alcoholism and/or of dependence on the intake of substances having an addictive potential.

20. The use according to claim 19, wherein the vicinal dithioglycol is Unithiol (DMPS).
